# NEW EUROPEAN PATENT SPECIFICATION

(11) **EP 0 261 835 B2**
(45) Date of publication and mention of the opposition decision: **21.05.1997**
(45) Mention of the grant of the patent: 16.06.1993
(21) Application number: 87307953.7
(22) Date of filing: 09.09.1987
(51) Int. Cl.: A61M 5/14

(54) **Improved winged catheter assembly**
Kathetersatz mit Flügeln
Ensembles cathéters à ailes

(30) Priority: 10.09.1986 US 905597
(43) Date of publication of application: 30.03.1988
(73) Proprietor: CRITIKON, INC., Tampa Florida 33607 (US)
(72) Inventor: Kvalo, Michael M., Conyers GA 30208 (US)
(74) Representative: Jones, Alan John

(56) References cited:
- EP-A- 0 066 667
- DE-B- 2 934 713
- FR-A- 2 242 114
- US-A- 4 059 105
- US-A- 4 170 993
- US-A- 4 194 504
- US-A- 4 392 856
- US-A- 4 460 356
- Der "VICRA Quik-Cath-Venenkatheter" von TRAVENOL GmbH

## Description

This invention relates, in general, to catheter assemblies and, more particularly, to catheter assemblies provided with means for anchoring the same to a patient, after the catheter has been emplaced, to prevent unwanted movement. Most specifically, this invention relates to so-called winged catheters.

Catheter assemblies are exemplified by the over-the-needle intervascular catheter assembly which is provided to insert a hollow catheter into a blood vessel as a means whereby fluids may be introduced into the blood stream of a patient. In such assemblies, a catheter extends distally and coaxially from a catheter hub, the hub having a lumen aligned with that of the catheter. A needle is provided, extending through the catheter hub, and through the lumen of the catheter, with a sharpened end of said needle projecting distally from the distal end of the catheter. In use, the needle is employed to pierce the skin and enter the blood vessel of the patient. The catheter is urged distally into the blood vessel and the needle is then urged proximally out of the assembly. With the needle removed, means for providing the fluid to be introduced into the blood vessel is affixed to the catheter hub so as to be in flow communication with the catheter lumen, whereafter the infusion of such fluid can begin.

It is important that at some point in this process, the catheter hub be firmly secured to the patient in that the infusion process is frequently carried out for long periods of time and there is the danger of dislodging the catheter from the blood vessel unless movement of the catheter hub relative to the patient is inhibited. While in some instances this securement is accomplished by very positive means such as suturing, most frequently pressure sensitive adhesive tape is employed. Generally, the catheter is first emplaced and then taped to the patient's body by applying a strip or strips of adhesive tape across the hub.

It has been suggested that the taping and securement of the catheter hub to the body of the patient can be facilitated if extended surfaces or wings are provided, extending from the catheter hub transversely to the direction of the axis of the hub. Adhesive tape may then be applied to these wing-like surfaces. Additionally, these wings prevent axial rotation of the hub and catheter after emplacement. Examples of suggestions for such winged catheters may be found in U. S. Patents 3,064,648 to A. F. Bujan; 3,352,306 to S. Hirsch; 3,589,361 to D. A. Loper et al.; 3,714,945 to V. F. Stanley; 3,769,975 to M. Nimoy et al.; 4,192,304 to M. J. Millet; 4,192,305 to C. H. Seberg; 4,193,399 to T. P. Robinsin; 4,194,504 to J. L. Harms et al.; 4,300,553 to C. H. Seberg; 4,389,210 to J. W. Genese; 4,392,856 to J. Lichtenstein: 4,366,817 to J. J. Thomas; and Des 257,885 to L. K. Kulle; as well as published U. K. Patent Application GB 2088215 to H. G. Wallace, Ltd.

In each of the above set out prior suggestions, a winged catheter is disclosed which wings may be used as surfaces for taping the hub and catheter to a patient. Typical of such securement is the method shown in the above mentioned U. S. Patent 4,366,817. Here a catheter is provided with wings extending from the catheter hub. Each wing has a proximal edge and a distal edge essentially parallel to each other and essentially perpendicular to the axis of the catheter assembly. Two strips of pressure sensitive adhesive tape are employed to secure this assembly to the patient, such strips being applied to extend, on either side of the catheter assembly, parallel to the axis of the catheter and overlying the portions of the wings remote from the catheter hub. This configuration of adhesive tape, i.e., parallel to the catheter, provides excellent securement for the assembly once it is so applied. Unfortunately, in actual hospital practice, this application is quite difficult to accomplish. It must be recognized that, firstly, one hand of the nurse or doctor is occupied with holding the assembly in place while with the other hand, the nurse attempts to apply the pressure sensitive adhesive tape, smoothly and securely, to the patient's arm. It will be appreciated that the adhesive tape has the unfortunate property of folding over on itself and hence is not easily applied with one hand. Further, once applied, adjustment of the tape is only possible at the expense of patient discomfort.

Before the priority date of the present Patent, Travenol GmbH made available a catheter placement assembly comprising: a catheter hub having a hub axis; a catheter extending distally from the catheter hub and being generally coaxially aligned with the hub axis; and a pair of opposed wings integral with the hub and extending from the hub in a direction transverse to the hub axis, wherein a portion of the proximal edge of each wing adjacent the hub forms an acute angle with the hub axis. The Travenol catheter placement assembly was secured to a patient using strips of adhesive tape extending distally parallel to the hub axis.

There is a need for a catheter placement assembly and technique for securing same to a patient in a more convenient manner than prior art suggestions.

The present invention provides a catheter placement assembly as defined in claim 1. Preferred features of the assembly are set forth in the dependent claims. The catheter placement assembly of the present invention, in conjunction with a specific method of applying adhesive tape, may be anchored to a patient after catheter insertion without encountering the drawbacks of prior art assemblies. Specifically, the catheter placement assembly of this invention allows the nurse to hold the catheter placement assembly with one hand and to neatly and efficiently apply anchoring adhesive tape with the other hand.

It has been discovered that, with the wing edges provided within the narrowly defined angular range given in claim 1, the method of taping described herein may be employed by a nurse without untoward difficulty.

Specifically, the method to be employed with the catheter assembly of this invention is as follows: Firstly, prior to commencing the emplacement of the catheter assembly, the nurse applies two strips of one-sided pressure sensitive adhesive tape with the adhesive side against that surface of the wings intended to be placed against the patient's body. The strips are applied essentially perpendicularly to the axis of the catheter and are of sufficient length to extend beyond the remote ends of each of the wings. The catheter is then inserted using normal insertion procedures. Because the non adhesive side of the tape is against the body, the tape does not interfere with the necessary movement of the catheter assembly relative to the body while the catheter is being emplaced. Having now emplaced the catheter, the nurse may place one hand on the hub to hold the assembly firmly against the patient. With the other hand the nurse may grip one extended end of one of the tapes and fold the tape over one angular edge of the wing so that the adhesive side now faces the patient. Because of the critically narrow range of angularity chosen for the angular edge, the tape will now extend approximately parallel to the catheter axis. The nurse need only press the tape firmly into contact with the patient, repeat the procedure with the other end of the tape and then repeat the procedure with the other strip of tape.

The catheter assembly is now firmly anchored to the patient so that the nurse may release the catheter hub. Preferably to insure securement, a third strip of adhesive tape is applied, adhesive side toward the patient, overlying the hub and wings and extending transversely to the axis of the catheter, on either side of the wings. The extended portion of this third strip is adhered to the patient. Accordingly, the catheter assembly is secured in both the transverse and the axial directions.
Fig. 1 illustrates, in perspective view, an embodiment of a catheter assembly;
Fig. 2 is a cross-sectional planar view of the catheter assembly of Fig. 1 taken along lines 2-2;
Fig. 3 is a transverse cross-section view of the catheter assembly of Fig. 1 taken along line 3-3;
Fig. 4 illustrates, in perspective view, an embodiment of a catheter assembly anchored to a patient's body with adhesive tape;
Fig. 4A is a partial planar view of the catheter assembly of Fig. 1 after a first step in the process of applying the anchoring adhesive tape;
Fig. 4B is a partial planar view of the catheter assembly of Fig. 1 after a further step in the process of applying the anchoring tape;
Fig. 4C is a partial planar view of the final step in the process of applying the anchoring tape;
Fig. 5 is an embodiment of the catheter assembly of the present invention shown in a view similar to that of Fig. 4b.

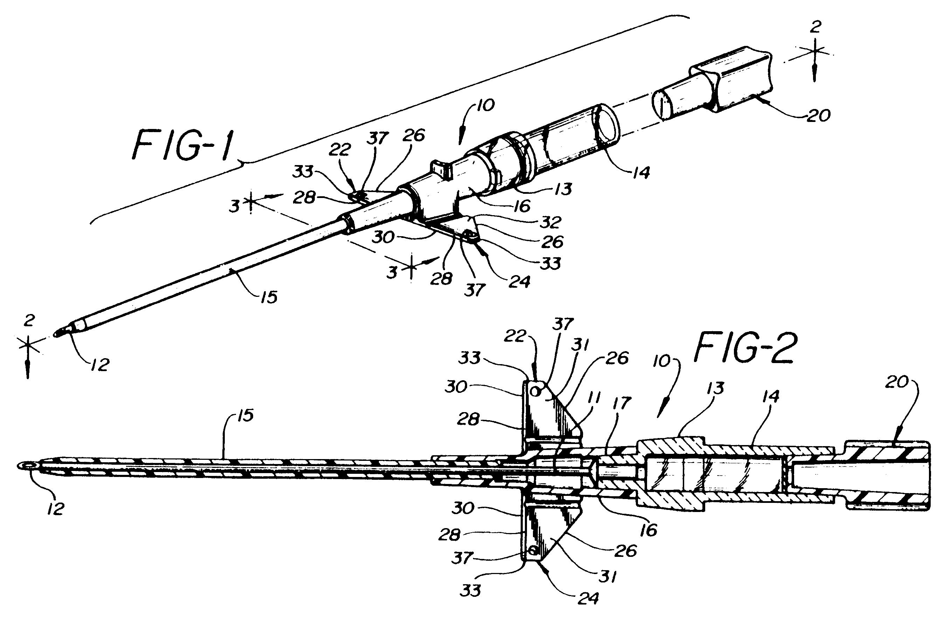

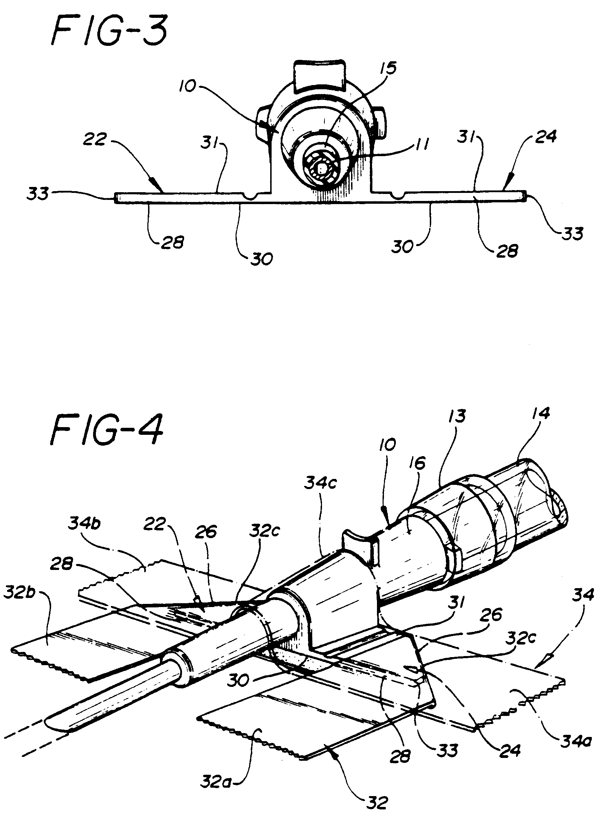

Referring to Figures 1-4, illustrated therein is a catheter assembly 10, which is described in order to explain the taping thereof to a patient. The assembly comprises an introducer needle 11 which is in the form of a hollow hypodermic needle having a point 12 on one end thereof. Needle 11 is secured at its blunt end to a needle hub 13 which has a transparent blood detecting chamber 14 integral with its proximal end. The entire hub and blood detecting chamber assembly may preferably be molded in one piece from a suitably clear plastic material, e.g. polypropylene, polyacrylate, polycarbonate, or styrene butadiene copolymer. Needle 11 serves the function of introducing a flexible polymeric catheter 15 into a blood vessel. Catheter 15 is attached to a catheter hub 16 at its proximal end and hub 16 is adapted to be removably secured to a fitting 17 on the distal end of needle hub 13. A plug 20 is provided for insertion into the proximal end of blood detecting chamber 14 to close such chamber against the passage of blood therefrom.

To emplace the catheter 15, the assembly as shown in Fig. 1, with the needle 11 and plug 20 in place, is employed. The needle 11, with its protruding end 12, is used to pierce the skin and blood vessel of the patient. Thereafter, the catheter 15 is urged distally to be seated in the blood vessel and the needle is urged proximally to be removed from the assembly together with the needle hub 13 and plug 20. A fluid administration means may now be placed in flow communication with the catheter hub 16 and the catheter 15.

As described above, at some point in this process, it is necessary to secure the catheter and catheter hub to the patient, preferably by the use of adhesive tape. To this end the assembly 10 has been provided with wings 22 and 24, extending from the catheter hub 16 in a direction transverse to the longitudinal axis of the coaxial catheter hub and catheter. Each of said wings 22 and 24 comprise proximal edges 26, distal edges 28, a body facing surface 30, a top surface 31 and extreme ends 33. The wings 22 and 24 provide a convenient surface for applying the adhesive tape and may also be used for anchoring the assembly by suturing, employing suture holes 37.

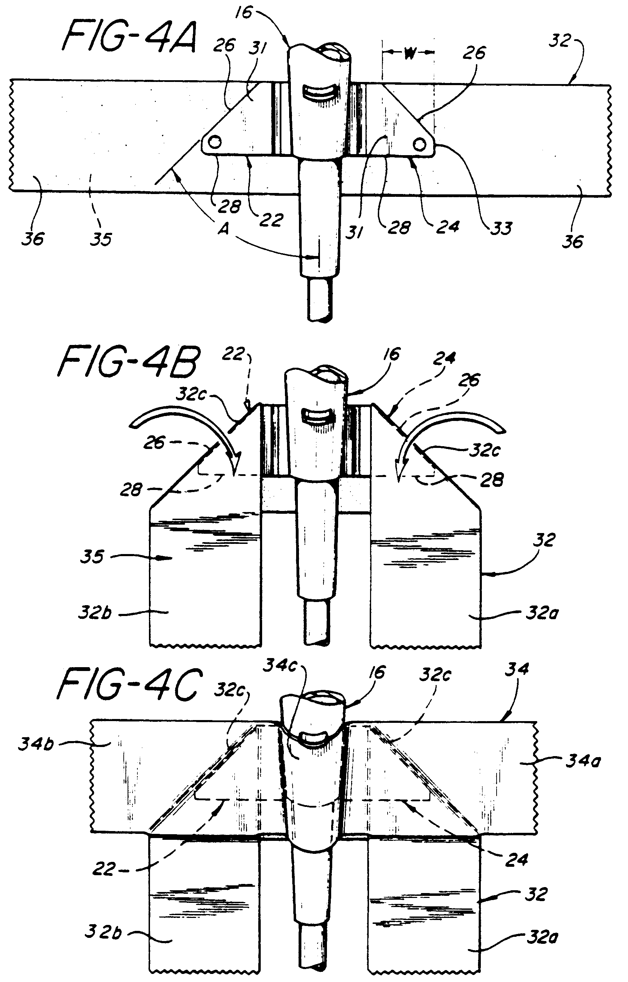

Additionally, the wings 22 and 24 are each provided with hinges or notched areas 35, to facilitate the planar wings conformance with the surface of the patient. A portion of one straight edge, e.g., the proximal edge 26 in the embodiment shown in Figs. 1-3, is angular, forming an acute angle with the longitudinal axis of the coaxial catheter-catheter hub assembly. The acute angle, angle A in Fig. 4A, may range from about 35° to about 55° and, preferably, from about 40 to 50°, e.g. 45°. The portion of the angular edge which is at the above prescribed angle should be sufficient so that the transverse projected length of this portion, dimension W in Fig. 4A, is approximately at least as long as a substantial fraction of the width of the strip of adhesive tape being employed, e.g. about at least one third of the width of such adhesive tape. Preferably, this transverse projected length W should be in the range 3.17-19.05 mm (0.125-0.75 inches) and still more preferably 3.81-13.10 mm (0.15-0.25 inches) e.g. 4.83 mm (0.19 inches).

The importance of the above prescribed geometric relationships will become apparent from consideration of Figures 4 and 4a-c, wherein the taped catheter assembly as well as the taping steps are illustrated.

Fig. 4 illustrates the catheter assembly securely taped to the body of a patient (not shown). A first strip of adhesive tape 32 is adhered to the patient's body with the first tape strip legs 32a and 32b extending essentially parallel to the axis of the catheter and with the central portion 32c adhered to the body facing surface of the catheter hub 16 and wings 22 and 24. A second strip of adhesive 34 is applied with the central portion thereof 34c being adhered to the top of the hub 16 and the top surface of the wings 22 and 24 and the second tape strip legs 34a and 34b extending beyond the ends of the wings and being adhered to the patient.

Figs. 4A-C illustrate the steps employed in achieving the secured configuration shown in Fig. 4. Referring to Fig. 4, prior to inserting the catheter into the patient, the user first applies first tape strip 32 to the catheter with the central portion of the tape 34c being adhered to the bottom or body side of the catheter hub and the body side surface of the wings. The first tape strip legs 32a and 32b then extend beyond the ends of the wings with their adhesive coated surfaces 36 facing upwardly. Accordingly, the catheter may be inserted into the patients blood vessel and the applied first adhesive tape strip 32 will not interfere as it is the non-adhesive coated surface 35 that contacts the patient.

After insertion (referring to Fig. 4B) the nurse, holding the catheter assembly in place with one hand, may now simply grip the remote ends 36 of each of legs 32b and 32a, and fold these portions of tape strip 32 over the wings 22 and 24 and parallel to the axis of the catheter-catheter hub. It can be seen that by providing an angular portion for proximal edge 26, within the narrow range of about 35-55°, legs 32a and b will be approximately parallel to the axis of the assembly and fold neatly over the wings. This is facilitated by having the projected length of the angular portion W in Fig. 4A, dimensioned to a length essentially at least that of the width of the tape strip 32.

Referring to Fig. 4C, having now secured the catheter assembly to the patient, the nurse may now release the same and for added securement, apply a second adhesive tape strip 34. This adhesive strip may be applied adhesive side down, with its central portion 34c overlying the catheter hub and adjacent wing surfaces and with legs 34a and 34b, extending beyond the wings onto the patient's body.

The embodiment illustrated in Figs. 1-4, and 4A-C comprises certain preferred features. For example the angular edge is chosen to be the proximal edge 26 of the wings so that the tape legs 32a and 32b will extend toward the catheter and away from potentially encumbering apparatus such as the liquid administration set or other devices to be employed in conjunction with the catheter. Further, the angular portion is on the part of the wings most remote from the catheter hub giving the nurse maximum access to maneuver the tape. Nevertheless, in certain circumstances other variations may be more appropriate.

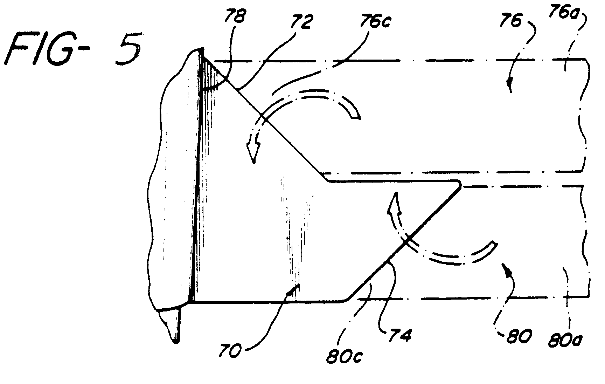

The wing shape illustrated in Fig. 5, provides an embodiment according to the present invention wherein both the adjacent portion of the proximal edge 72 of wing 70 and the remote portion of distal edge 74 are angular. This embodiment allows for an advantageous method of applying two strips of adhesive tape to the patient with use of only one hand. Specifically, prior to emplacing the catheter assembly, the nurse applies a first strip 76 with the adhesive side of the central portion of the strip 76c adhered to the proximal portion of the bottom (patient facing side) of the catheter hub 78 and the wings 70 and with the leg portions of strip 76 (leg 76a being illustrated in Fig. 5) extending beyond the wings. Similarly, a second strip 80 is applied with the adhesive side of the central portion of the strip 80c adhered to the distal portion of the bottom (patient facing side) of the catheter hub 78 and the wings 70 and with the leg portions of strip 80 (leg 80a being illustrated in Fig. 5) extending beyond the wings.

The catheter assembly shown in Fig. 5 is emplaced, and again, because the adhesive surfaces of strip 76 and 80 are face up, the tape does not interfere with placement. Once emplaced the nurse may now hold the catheter against the patient with one hand and with the other grip a first remote end of strip 76 and fold the strip over proximal edge 72, parallel to the axis of the catheter and in the distal direction. Next, without releasing the catheter assembly, the nurse may grip the second remote end of strip 76 and also fold the strip over the proximal edge 72 parallelly and in the distal direction. Then, again without releasing the catheter, the nurse may grip a first remote end of strip 80 and fold the strip over the distal edge 74 of the wing, parallel to the axis of the catheter and, this time, extending in a proximal direction. Finally, still without releasing the catheter, the nurse may fold the second remote end of tape 80 in a similar fashion.

## Claims

1. A catheter placement assembly comprising:
a catheter hub (16) having a hub axis;
a catheter (15) extending distally from the catheter hub (16) and being generally coaxially aligned with the hub axis; and
a pair of opposed wings (70) integral with the hub and extending from the hub in a direction transverse to the hub axis,
wherein:
a portion of the proximal straight edge (72) of each wing forms an acute angle with the hub axis of 35 to 55 degrees;
a portion of the distal straight edge (74) of each wing forms an acute angle with the hub axis of 35 to 55 degrees; and
one of said proximal and distal angled portions is toward the hub and the other is remote from the hub,
whereby the taping of the hub to a patient is facilitated.

2. The assembly of claim 1, wherein said portion of said proximal edge (72) of each wing forms an acute angle with the hub axis of 40 to 50 degrees.

3. The assembly of claim 1 or claim 2, wherein the transverse projected length of said portion of said proximal edge (72) of each wing is at least 3.17 mm.

4. The assembly of any one of claims 1 to 3, further including two strips of adhesive tape (76, 80) extending transversely of the hub across the wings, one strip of tape (76) extending over said portion of said proximal edge (72) and the other strip of tape extending over said portion of said distal edge (74), wherein the transverse projected length of each of said portions is at least a substantial fraction of the width of the respective strip of adhesive tape.

5. The assembly of claim 5, wherein the substantial fraction is one third.

## Patentansprüche

1. Katheteransetzeinrichtung, mit:
einer Katheterbuchse (16) mit einer Buchsenachse;
einein Katheter (15), der sich distal von der Katheterbuchse (16) aus erstreckt und allgemein koaxial mit der Buchsenachse ausgerichtet ist; und
einem Paar einander gegenüberliegender Flügel (70), die mit der Buchse einstückig sind und sich von der Buchse in einer Richtung quer zur Buchsenachse erstrecken,
wobei:
ein Abschnitt der proximalen geraden Kante (72) jedes Flügels mit der Buchsenachse einen spitzen Winkel von 35 bis 55° bildet;
ein Abschnitt der distalen geraden Kante (74) jedes Flügels mit der Buchsenachse einen spitzen Winkel von 35 bis 55° bildet; und
einer der genannten proximalen und distalen Schrägabschnitte gegen die Buchse liegt und der andere von der Buchse entfernt liegt,
wodurch das Anbringen der Buchse mittels eines Bandes am Patienten erleichtert ist.

2. Vorrichtung nach Anspruch 1, bei welcher der genannte Abschnitt der proximalen Kante (72) jedes Flügels mit der Buchsenachse einen spitzen Winkel von 40 bis 50° bildet.

3. Vorrichtung nach Anspruch 1 oder 2, bei welcher die in Querrichtung projizierte Länge dieses Abschnittes der proximalen Kante (72) jedes Flügels zumindest 3,17 mm beträgt.

4. Vorrichtung nach einem der Ansprüche 1 bis 3, ferner mit zwei Selbstklebebandstreifen (76, 80), die sich quer zur Buchse über die Flügel erstrecken, wobei ein Selbstklebebandstreifen (76) sich über den genannten Abschnitt der proximalen Kante (72) erstreckt und der andere Selbstklebebandstreifen sich über den genannten Abschnitt der distalen Kante (74) erstreckt, wobei die in Querrichtung projizierte Länge jedes der genannten Abschnitte zumindest ein wesentlicher Bruchteil der Breite des jeweiligen Selbstklebebandstreifens ist.

5. Vorrichtung nach Anspruch 4, bei welcher der wesentliche Bruchteil ein Drittel ist.

## Revendications

1. Un ensemble de mise en place de cathéter comprenant:
un moyeu de cathéter (16) ayant un axe de moyeu;
un cathéter (15) s'étendant à distance du moyeu de cathéter (16) et étant généralement aligné en position coaxiale par rapport à l'axe du moyeu; et
une paire d'ailes opposées (70) faisant partie intégrante du moyeu et s'étendant à partir du moyeu dans une direction transversale par rapport à l'axe du moyeu,
dans lequel
une partie du bord droit proximal (72) de chaque aile forme un angle aigu de 35 à 55 degrés par rapport à l'axe du moyeu;
une partie du bord droit distant (74) de chaque aile forme un angle aigu de 35 à 55 degrés par rapport à l'axe du moyeu; et
l'une desdites parties en angle proximale et distante est en direction du moyeu et l'autre est éloignée du moyeu,
ce qui facilite le collage du moyeu sur un patient.

2. L'ensemble selon la revendication 1, dans lequel ladite partie dudit bord proximal (72) de chaque aile forme un angle aigu de 40 à 50 degrés par rapport à l'axe du moyeu.

3. L'ensemble selon la revendication 1 ou 2, dans lequel la longueur transversale projetée de ladite partie dudit bord proximal (72) de chaque aile est d'au moins 3,17 mm.

4. L'ensemble selon l'une des revendications 1 à 3, comprenant en outre deux bandes de ruban adhésif (76, 80), s'étendant transversalement à partir du moyeu et en travers des ailes, une bande de ruban (76) s'étendant par dessus ladite partie dudit bord distant (74), dans lequel la longueur transversale projetée de chacune desdites parties est au moins une fraction substantielle de la largeur de la bande respective de ruban adhésif.

5. L'ensemble selon la revendication 4, dans lequel la fraction substantielle est un tiers.
